# FASCICULE DE BREVET EUROPEEN

(11) **EP 2 787 980 B1**
(45) Date de publication et mention de la délivrance du brevet: **24.10.2018**
(21) Numéro de dépôt: 12795478.2
(22) Date de dépôt: 06.12.2012
(51) Int. Cl.: A61K 9/20

(54) **COMPRIME SUSCEPTIBLE DE LUTTER CONTRE LE DETOURNEMENT PAR VOIE INJECTABLE**
TABLETTE GEGEN MISSBRAUCH DURCH INJEKTION
TABLET CAPABLE OF COMBATTING MISUSE BY INJECTION

(30) Priorité: 06.12.2011 FR 1161249
(43) Date de publication de la demande: 15.10.2014
(73) Titulaire: Ethypharm, 92210 Saint-Cloud (FR)
(72) Inventeur: HERRY, Catherine, F-27670 Saint-Ouen du Tilleul (FR); CONTAMIN, Pauline, F-76220 La Feuillie (FR)
(74) Mandataire: Regimbeau
(86) Numéro de dépôt international: PCT/EP2012/074671
(87) Numéro de publication internationale: WO 2013/083710

(56) Documents cités:
- WO-A1-03/013476
- WO-A1-2007/099152
- WO-A2-2007/000779
- US-A1- 2005 031 546

## Description

Le détournement ou le mésusage volontaire (ou encore, suivant le terme anglo-saxon de "*drug-abuse*") qualifie l'utilisation détournée à des fins toxicomanes de certains médicaments, en particulier de certains agents psychotropes ou stupéfiants, tels que les opioïdes ou leurs dérivés utilisés en thérapeutique dans le traitement des douleurs sévères ou dans le traitement de l'addiction aux drogues opiacées.

Dans le cas d'un détournement par injection le comprimé ou la gélule contenant les principes actifs d'intérêt est réduit en poudre fine à l'aide de tous les moyens possibles à la disposition du toxicomane notamment à l'aide d'un mortier ou d'un briquet voire par simple mastication ou en croquant le comprimé. La poudre grossière obtenue, qui contient nécessairement les excipients initialement présents dans la forme galénique peut alors être dissoute dans un faible volume de liquide (quelques millilitres) parfois préalablement chauffé et/ou additionné d'acide dans le cas de certains actifs présents sous leur forme base (héroïne brune, morphine base). Le liquide obtenu peut ensuite être filtré sommairement pour limiter l'introduction dans le sang de grosses particules, à l'aide d'un filtre à cigarette par exemple avant d'être injecté par voie intraveineuse.

Dans ce cas, le principe actif est immédiatement disponible dans le sang, procurant l'effet psychotrope immédiat recherché par le toxicomane.

Le détournement s'accompagne en outre de nombreux risques sanitaires liés directement à l'injection des excipients et des résidus de broyage non purifiés, peu ou mal filtrés et non stériles.

Il est connu de l'état de la technique la demande WO2007099152. Cette demande porte sur des comprimés matriciels à libération prolongée, insolubles dans l'eau et ultra-durs. Ces comprimés matriciels comprennent un principe actif dispersé au sein d'une matrice de compression constituée d'au moins un excipient choisi dans le groupe comprenant les polymères retard pH indépendants et insolubles dans l'eau, les excipients minéraux et leurs mélanges.

De tels comprimés permettent d'éviter les phénomènes de mésusage involontaire lorsque le comprimé est pris en présence d'alcool ou lorsqu'il est croqué accidentellement par le patient. Les comprimés décrits dans WO2007099152 permettent également d'éviter le mésusage volontaire en empêchant le détournement d'actif par voie orale et nasale.

Toutefois, ces comprimés ne permettent pas d'empêcher le détournement d'actif par voie injectable.

Il est également connu de l'état de la technique le document US 4,070,494. Ce document porte sur des compositions pharmaceutiques qui évitent le détournement de principe actif par voie parentérale en limitant l'extraction d'actif en présence d'eau. Ces compositions comprennent un excipient qui gélifie dans l'eau.

Toutefois, dans ce document aucune solution n'est apportée pour éviter l'extraction d'actif en milieu acide ou hydroalcoolique.

On connait également l'Oxycontin®, comprimé d'oxycodone largement détourné par les toxicomanes. Sa formulation a été revue pour limiter les abus. Cependant, même après reformulation, le détournement par voie injectable est toujours possible.

Il existe donc un besoin réel pour la mise au point d'une composition pharmaceutique qui rende mal aisée voire impossible l'extraction de principe actif dans tous milieux liquides qu'ils soient aqueux (quel que soit le pH) ou hydro-alcoolique, notamment dans l'acide chlorhydrique, le vinaigre, le jus de citron, l'éthanol à 40%, l'éthanol à 50%, l'éthanol à 60%, l'éthanol à 70%, l'éthanol à 80%, l'éthanol à 90% et l'éthanol à 96%.

Cette forme pharmaceutique doit par ailleurs être fabriquée au moyen d'un procédé de fabrication extrêmement simple, rapide et peu coûteux.

Le but de la présente invention est donc de proposer une composition pharmaceutique orale solide à libération prolongée pour son utilisation comme médicament pour lutter contre le détournement par voie injectable.

Ainsi l'invention a pour objet une composition pharmaceutique orale sous forme de comprimé à libération prolongée comprenant :
- au moins un principe actif susceptible d'être détourné à des fins de toxicomanie,
- un mélange d'acétate de polyvinyle et de polyvinylpyrrolidone,
- de la Gomme xanthane,
- un carbomère.

### DEFINITIONS

Dans la présente invention, on entend par « libération prolongée », la libération d'un ou plusieurs principes actifs dans l'organisme pendant des périodes supérieures à 6 heures, plus préférentiellement supérieures à 8 heures, voire sur une période supérieure à 24 heures, rendant possible une administration de ces actifs soit en deux prises par jour, soit en une seule prise par jour.

Dans le cadre de la présente invention les termes mésusage volontaire, détournement ou *drug-abuse* sont employés pour désigner toute altération intentionnelle des formes galéniques. En particulier, la notion de mésusage volontaire concerne la réduction sous forme de poudre de comprimés puis leur dissolution dans une faible quantité de liquide en vue d'une injection parentérale.

Préférentiellement, on entend par « lutter contre le détournement par voie injectable » que la quantité de principe actif extrait dans un milieu hydro-alcoolique est inférieure à celle de l'Oxycontin®, c'est-à-dire inférieure à 40% d'actif extrait. La quantité de principe actif extrait dans un milieu aqueux est inférieure à celle de l'Oxycontin®, c'est-à-dire inférieure à 25 % d'actif extrait.

On entend par « solution aqueuse » tout milieu liquide, quel que soit son pH : acide, neutre ou basique, contenant au moins une partie d'eau. On peut citer comme exemples l'eau, le vinaigre, le jus de citron, les sodas, etc.

Dans la présente invention, l'expression « solution hydro-alcoolique» est définie comme tout milieu liquide contenant au moins une partie d'eau et une partie d'éthanol tels que l'éthanol à 40%, l'éthanol à 50%, l'éthanol à 60%, l'éthanol à 70%, l'éthanol à 80%, l'éthanol à 90% et l'éthanol à 96%.

On entend par « carbomère » ou « Carbopol® » un polymère d'acide acrylique de haut poids moléculaire réticulé avec du sucrose allylique ou des éthers allyliques de pentaérythritol (handbook of Pharmaceutical Excipients, 5^{ème} Edition, p111). Par exemple, il s'agit du carbomère 910, du carbomère 934, carbomère 934P, du carbomère 940, du carbomère 941, du carbomère 71G, du carbomère 980, du carbomère 971P ou du carbomère 974P. La viscosité dudit carbomère est comprise entre 4000 et 60000 cP à 0,5% w/w.

Dans le cadre de la présente invention, on entend par « comprimé » des comprimés matriciels, des comprimés multicouches, des micro-comprimés.

### DESCRIPTION

La composition conforme à l'invention peut contenir un ou plusieurs principes actifs susceptibles d'être détournés qui peuvent être de toute nature. On choisira avantageusement des principes actifs destinés à être libérés dans l'organisme de façon prolongée, c'est à dire sur des périodes de temps d'au moins 6 heures et préférentiellement supérieures à 12 heures, plus préférentiellement encore, supérieures à 20 heures.

Ainsi, les comprimés conformes à l'invention sont préférentiellement employés pour la délivrance prolongée d'actifs appartenant à la famille des agents psychotropes, c'est à dire capables d'agir sur le psychisme par des effets stimulants, tranquillisants ou hallucinogènes.

Ainsi, les principes actifs utilisables dans le cadre de l'invention sont préférentiellement des dérivés et/ou des alcaloïdes de l'opium, naturels ou de synthèse tels que la codéine, la narcéine, la noscapine et leurs sels.

Les actifs utilisables selon l'invention appartiennent en outre au groupe comprenant la morphine, ses dérivés et leurs sels et en particulier les morphinènes tels que la pholcodine, la nalorphine, la codéine, l'hydrocodone, la pholcodine, la dihydrocodéine, l'hydromorphone, et les morphinanes tels que la buprenorphine, le butorphanol, le dextromethorphane, la nalbufine, la naltrexone, la naloxone, le nalmefene, l'hydrocodone, l'oxymorphone et l'oxycodone et d'une manière générale tous les analogues de la morphine et tous les analgésiques morphiniques, tels que le fentanyl, l'alfentanyl, le sufentanyl, le tramadol, l'apomorphine et l'étorphine.

La présente invention s'adresse également aux dérivés d'alcaloïdes, naturels ou de synthèse ayant un effet psychotrope, tels que la cocaïne et ses dérivés, l'héroïne, le cannabis et les cannabinoïdes.

Enfin, la présente invention s'adresse également à toutes les substances utilisées actuellement en thérapeutique comme traitement de substitution aux addictions et l'aide au sevrage, telles que la méthadone et la buprénorphine par exemple, très sujettes au détournement.

D'une manière générale, la présente invention est également envisageable pour toutes les autres classes thérapeutiques de médicaments actuellement sujettes au détournement, et notamment les antipsychotiques, les tranquillisants, les hypnotiques, les analgésiques, les anxiolytiques, en particulier la classe des benzodiazépines, les amphétaminiques.

Le ou les principe(s) actif(s) présent(s) dans la composition représente(nt) entre 1 et 70% en poids du poids total de la composition. Avantageusement le ou les principe(s) actif(s) représente(nt) de 5 à 50% en poids du poids total de la composition et encore plus avantageusement de 10% à 40% en poids du poids total de la composition.

Il est également possible de quantifier le principe actif en milligrammes (mg). Ainsi, le ou les principe (s) actif(s) présent(s) dans la composition représente(nt) entre 0,1 mg et 500 mg dans le comprimé. Avantageusement le ou les actifs représente (nt) de 1 mg à 200mg dans le comprimé. Par exemple, un comprimé d'oxycodone comprend de 5 à 80 mg d'oxycodone.

Par exemple, un comprimé d'hydromorphone comprend de 4 à 64 mg d'hydromorphone et un comprimé de morphine de 5 à 200 mg de morphine.

Le ou les principes actifs contenu(s) dans la composition selon l'invention peuvent être présents sous toute forme connue de l'homme du métier, notamment sous forme de poudre, de cristaux ou de granules.

La composition conforme à l'invention contient également un mélange d'acétate de polyvinyle et de polyvinylpyrrolidone. Le ratio d'acétate de polyvinyle et de polyvinylpyrrolidone dans le mélange est compris entre (95:5) et (70:30), de préférence il est de (80:20).

Le mélange d'acétate de polyvinyle (PVA) et de polyvinylpyrrolidone (PVP) représente de 10 à 80 % en poids du poids total de la composition, de préférence de 15 à 70 % en poids du poids total de la composition et encore plus préférentiellement de 20 à 60 % en poids du poids total de la composition.

La gomme xanthane est également présente dans la composition selon l'invention. Elle représente de 1 à 88 % en poids du poids total de la composition, et plus particulièrement de 3 à 50 % en poids du poids total de la composition, de préférence 10 à 40 %, de manière encore préférée 5 à 30 %, de manière particulièrement préférée 15 à 25%.

La composition conforme à l'invention contient en outre un carbomère (Carbopol®). Les quantités de carbomère représentent de 1 à 88 % en poids du poids total de la composition, et plus particulièrement de 2 à 50%, de préférence de 5 à 40%, de manière encore préférée 8 à 30%, de manière particulièrement préférée de 10 à 30% en poids du poids total de la composition.

De façon surprenante, les inventeurs ont découvert que l'association spécifique des composés décrits ci-dessus permet d'empêcher le détournement par voie injectable lorsque la forme pharmaceutique est réduite en poudre en vue d'être dissoute dans un volume injectable d'une solution aqueuse ou hydro-alcoolique.

En effet, lorsque la composition selon la présente invention est réduite sous forme de poudre puis mise en solution aqueuse (même pour des pH < 3) ou hydro-alcoolique, un gel se forme, piégeant l'actif et rendant l'administration par voie parentérale difficile.

De plus, à la vue de l'épaisseur du gel formé, le toxicomane sera fortement dissuadé de s'injecter une telle composition.

Par ailleurs, le fait que cette composition soit insoluble en milieu alcoolique ceci évite la libération massive de principe actif dans l'organisme lors d'une prise concomitante avec de l'alcool et sécurise le mésusage accidentel.

Un autre avantage de cette composition est qu'elle limite le détournement par voie nasale. En effet, lorsque la composition est réduite sous forme de poudre afin d'être absorbée par la voie nasale, un gel se forme au contact des sécrétions muqueuses, piégeant ainsi l'actif.

La composition pharmaceutique de la présente invention présente également une résistance à l'écrasement d'au moins 4 MPa, avantageusement d'au moins 6 MPa. Dans la présente demande, on emploiera à la fois la notion de résistance à l'écrasement et la notion de dureté telles que décrites dans la demande WO2007099152 pour caractériser les comprimés.

Grâce à sa résistance à l'écrasement, la composition de l'invention permet de limiter le détournement par voie orale. En effet, la composition présente une structure qui limite les risques de broyage par des techniques classiquement utilisées par les toxicomanes (écrasement sous une tasse ou entre deux cuillères) ou par mastication.

Selon un autre aspect de l'invention, les inventeurs ont également été surpris de constater que lorsqu'un agent modificateur de pH est ajouté à la composition selon la présente invention ; et lorsque la composition est réduite en poudre et mise en solution aqueuse ou hydro-alcoolique, le gel qui en résulte possède une viscosité telle que le gel ne peut plus passer à travers une aiguille de 20 gauge. Cette propriété est remarquable car elle éradique toute tentative d'administration par voie injectable.

L'agent modificateur de pH selon l'invention comprend au moins un composé suivant : acide citrique, bicarbonate de sodium, acide fumarique, phosphate de sodium, hydroxyde de Potassium, hydroxyde de sodium et carbonate de sodium.

Ainsi la présente composition pharmaceutique peut comprendre de 0,1 à 30% en poids du poids total de la composition d'agent modificateur de pH, de préférence de 0,5 à 20 % en poids du poids total de la composition d'agent modificateur de pH et encore plus préférentiellement de 1 à 10 % en poids du poids total de la composition d'agent modificateur de pH

Avantageusement, il a également été constaté que la présence d'agent modificateur de pH peut rendre la libération de l'actif pH-indépendante au cours de son trajet dans le tractus gastro-intestinal. Ainsi, la libération de l'actif peut ne pas être perturbée par les variations de pH des différents milieux.

Ladite composition pharmaceutique peut également comprendre au moins une des substances (a) à (e) suivante ou leur mélange :
- (a) une substance qui irrite les voies nasale et/ou pharyngée,
- (b) un antagoniste du ou des principe(s) actif(s) susceptible d'être détourné à des fins de toxicomanie,
- (c) une substance émétique,
- (d) un colorant comme agent aversif
- (e) une substance au goût amer.

Lorsque le principe actif est un dérivé opiacé naturel ou de synthèse, l'antagoniste sera avantageusement choisi dans le groupe comprenant la naloxone, la naltrexone, le nalmefene, le nalid, la nalmexone, la nalorphine et la naluphine, ces différents composés étant chacun sous une forme pharmaceutiquement acceptable, en particulier sous forme de base, de sel ou sous forme solvatée. Ces antagonistes sont présents à des doses classiquement utilisées, notamment à raison de 0,5 à 100 mg par composition.

Dans un mode avantageux de réalisation de l'invention, ledit agent antagoniste est la naloxone ou l'un de ses sels pharmaceutiquement acceptable.

Selon un mode particulier de l'invention, la composition peut être pelliculée à l'aide d'un enrobage externe que l'homme de l'art saura adapter en fonction des besoins et de la fonction attribuée à cet enrobage.

Ainsi, l'enrobage externe peut être appliqué à des fins de protection du principe actif, lorsqu'il s'agit d'un actif sensible aux pH faibles du milieu gastrique par exemple, on parlera alors d'enrobage gastrorésistant.

Par ailleurs, l'enrobage externe peut être appliqué pour ralentir encore la diffusion du principe actif. On pourra utiliser à cette fin, différents grades d'éthylcellulose ou de polymères méthacryliques bien connus de l'homme de l'art.

Enfin, l'enrobage externe peut être utilisé pour modifier l'aspect esthétique (texture, couleur) et/ou la palatabilité (sensation en bouche) de la composition pour le patient. En particulier, on pourra avantageusement utiliser des excipients tels que des dérivés cellulosiques ou des dérivés acryliques bien connus de l'homme de l'art pour masquer le goût du principe actif si nécessaire.

Un tel enrobage peut donc comprendre un mélange d'un ou plusieurs excipients connus de l'homme de l'art de nature différente, utilisés seuls ou en mélange pour les différentes fonctions énumérées plus haut.

Le ou les excipients utilisés pour l'enrobage sont appliqués de façon connue de l'homme du métier en quantité nécessaire pour obtenir la ou les fonctions recherchées.

Ces excipients peuvent être appliqués à la surface de la composition de façon classique par pulvérisation d'une solution ou suspension de l'agent d'enrobage dans un solvant, en turbine perforée ou en lit fluidisé par exemple.

La composition conforme à l'invention peut s'adapter à toutes formes galéniques, notamment elle peut se présenter sous forme de comprimé matriciel, de comprimé multicouche ou de micro-comprimés.

### Comprimé matriciel

On parle de « matrice » pour désigner une composition pharmaceutique dont la structure interne est homogène et identique du centre vers la périphérie de la composition.

La composition selon la présente invention est composée d'un mélange homogène de principe actif sous forme de poudre ou de granules et d'au moins un mélange d'acétate de polyvinyle et de polyvinylpyrrolidone associé à de la Gomme xanthane et du Carbopol.

Plus particulièrement, le(s) principe(s) actif(s) peu(ven)t être directement introduit(s) dans le mélange pour compression, monté(s) sur supports (obtention de microgranules) ou granulé(s) par voie humide ou sèche (obtention de granules).

Lorsque le ou les principe (s) actif(s) sont présents sous la forme de microgranules, ces microgranules peuvent être obtenus de façon classique par dépôt (montage) du ou des actifs à la surface de supports pharmaceutiquement neutres, tels que des microbilles préfabriquées à base de cellulose ou d'un mélange de sucre et d'amidon et vendues sous le terme *"sugar spheres"* ou encore des granulés d'autres excipients, comme le lactose par exemple.

Le procédé de dépôt (montage) de l'actif est réalisé de façon classique et connue de l'homme du métier et peut varier en fonction de la nature, de la quantité et de la fragilité du ou des principes actifs à déposer. Ainsi, le dépôt (montage) peut s'effectuer par pulvérisation d'une solution ou suspension du ou des principes actifs à la surface du support neutre ou la pulvérisation du ou des actifs en poudre à la surface du support préalablement humidifié à l'aide d'une solution d'agent liant.

Les granules de principe(s) actif(s) peuvent également être obtenus par granulation par voie sèche ou par voie humide du ou des principes actifs d'intérêt, généralement en présence d'au moins un agent liant et d'un liquide de mouillage le cas échéant, selon des techniques, là encore, bien connues de l'homme de l'art. Cette étape de granulation améliore l'uniformité de teneur des comprimés fabriqués.

Les granules ainsi obtenus sont mélangés avec :
- le mélange d'acétate de polyvinyle et de polyvinylpyrrolidone,
- la Gomme xanthane et,
- le Carbopol,
puis le mélange est comprimé.

Ainsi, la structure matricielle du comprimé selon l'invention, est extrêmement simple, ce qui rend sa fabrication industrielle aisée, puisqu'une simple étape de compression du mélange permet sa réalisation, sans qu'il soit nécessaire de chauffer les outils de compression et/ou le mélange avant ou pendant l'étape de compression proprement dite.

La matrice peut avantageusement, outre les excipients de la matrice contenir un ou plusieurs excipients destinés soit à favoriser le déroulement du processus de compression tels que des agents anti-collage comme la silice colloïdale, le talc, le stéarate de magnésium, le Polyéthylène Glycol (PEG) ou le stéarate de calcium, soit à améliorer la cohésion des comprimés lors de la compression, tels que les agents liants utilisés classiquement dans cette fonction, en particulier les amidons, les dérivés cellulosiques, soit des agents de charge, soit des lubrifiants, soit des plastifiants, soit des agents de remplissage, soit des édulcorants soit des colorants.

La dureté exceptionnelle des comprimés selon l'invention peut être obtenue sans qu'il soit nécessaire de faire subir au mélange pour compression (matrice de compression et principe actif) et/ou aux outils de compression (presse) une étape de chauffage préalablement ou pendant la compression.

Selon un autre mode de réalisation de l'invention, et lorsque sa granulométrie le permet, le principe actif est mélangé directement avec les excipients constituant la matrice de compression puis le mélange est directement comprimé.

Enfin, un autre mode possible de réalisation de l'invention consiste à mélanger le principe actif avec le ou les excipients de la matrice de compression, puis à granuler ce mélange par voie sèche ou humide afin d'obtenir des granules directement compressibles.

Les comprimés conformes à l'invention peuvent être de toute forme et de taille permettant d'obtenir des comprimés de dureté élevée.

La présente invention est donc adaptée à la fabrication tant de comprimés faiblement dosés en actif que de comprimés fortement dosés.

La présente invention concerne en outre le procédé de fabrication des comprimés matriciels conformes à l'invention. Ce procédé comporte les étapes suivantes :
- mélange du ou des principes actifs avec le ou les excipients de la matrice,
- éventuellement granulation et
- compression dudit mélange dans des conditions choisies de manière à ce que ledit comprimé présente une résistance à l'écrasement d'au moins 4 MPa, avantageusement d'au moins 6 MPa,
- éventuellement enrobage du comprimé.

La compression est réalisée sur machine à comprimer rotative. Les paramètres de compression doivent être choisis pour permettre de générer des comprimés de dureté adaptée à la présente invention. Toutefois, il n'est pas nécessaire de faire subir au mélange pour compression ou aux outils de compression une quelconque étape de chauffage avant et/ou pendant la compression dans le but d'atteindre l'exceptionnelle dureté observée sur les comprimés conformes à l'invention. Les forces de compression appliquées sont comprises entre 5 kN et 60 kN, avantageusement entre 10 kN et 30 kN. Elles sont choisies pour être compatibles avec le matériau des poinçons et pour être utilisables à cadences industrielles, tout en permettant d'obtenir des comprimés dont la résistance à la rupture est supérieure à 4 MPa, et préférentiellement supérieure à 6 MPa.

Lorsque le polymère d'enrobage du comprimé est un polymère retard, les comprimés enrobés conformes à l'invention peuvent avantageusement subir une phase de maturation dudit polymère d'enrobage afin de garantir sa stabilité physique et chimique. Cette étape est réalisée dans des conditions de température maîtrisée, inférieure à la température de fusion du principe actif pendant un temps contrôlé qui dépend du polymère d'enrobage et qui peut être compris entre 1 minute et plusieurs mois, pour un taux d'humidité relatif de 50 à 99%. Cette étape peut être réalisée en étuve ou en turbine.

### Les comprimés multi-couches

Pour pallier à d'éventuels problèmes de compatibilité chimique entre l'actif contenu dans la forme pharmaceutique et certains excipients de la formule ou entre deux actifs de la formule, la forme pharmaceutique peut être avantageusement une forme multicouche. Ainsi les composants qui ne sont pas compatibles chimiquement peuvent être séparés, par exemple la gomme xanthane peut être contenue dans une couche avec l'actif et le carbomère dans une autre couche.

Les propriétés de résistance à l'écrasement sont conservées et la forme pharmaceutique, dès lors qu'elle est réduite en poudre permet de limiter l'extraction dans les milieux aqueux / ou hydro-alcooliques de la même manière que la forme matricielle.

### Les micro-comprimés

On entend par « micro-comprimés » des comprimés de moins de 4 mm de diamètre.
L'invention a aussi pour objet une forme pharmaceutique orale comprenant des micro-comprimés à base de deux populations de micro-comprimés d'aspect extérieur identique. La première population (1) comprend au moins un agent gélifiant et au moins un principe actif pouvant créer une dépendance et la seconde population (2) comprend au moins un agent gélifiant de type carbomère. Cette forme pharmaceutique orale comprend des micro-comprimés dont l'utilisation abusive par écrasement puis injection ou inhalation n'est pas possible. Ces micro-comprimés peuvent être introduits à l'intérieur d'une gélule.
Une autre variante des micro-comprimés est d'associer deux populations constituées de composants identiques mais de faire varier les quantités de principe actif ainsi que les excipients et de mélanger ces deux populations de micro-comprimés.
Le procédé de fabrication des micro-comprimés est identique à celui décrit au paragraphe comprimé matriciel.

### EXEMPLES

### Exemple 1: Exemple comparatif dépourvu des éléments essentiels de l'invention de préparation d'un comprimé matriciel d'oxycodone HCl dosé à 40mg contenant de la gomme xanthane comme seul agent gélifiant mais pas de carbomère

| composants | mg/ comprimé | pourcentage |
|---|---|---|
| 1.Oxycodone granulé | 43.68 | 29.12 |
| 2.Kollidon SR® (PVA/PVP 80:20) | 75.27 | 50.18 |
| 3.Gomme Xanthane | 30.00 | 20.00 |
| 4.Syloïd | 0.30 | 0.20 |
| 5.Stéarate de magnésium | 0.75 | 0.50 |
| total | 150.00 | 100.00 |
| Dureté | 322 N | |
| Résistance à la rupture | 7.7 MPa | |

Dans l'exemple 1, les composants 1 à 4 sont tamisés sur 500µm puis mélangés au Turbula 10 min. Le stéarate de magnésium est ensuite ajouté et mélangé 1 min supplémentaire pour lubrification. Le mélange est ensuite comprimé directement sur presse rotative Sviac, équipée avec des poinçons ronds de diamètre 7mm, à une force de 16kN. La dureté qui en résulte correspond à une résistance à la rupture supérieure à 7MPa.

Pourcentages extraits dans différents milieux à partir des comprimés obtenus selon l'exemple 1

| Eau purifiée | HCl 0.1N | Ethanol 96% |
|---|---|---|
| 0% | 0% | 58% |

Lorsque le comprimé est broyé puis repris avec un volume de 10mL d'Ethanol 96%, la quantité d'oxycodone HCl recueillie après filtration est de 58% de la dose initiale. Par conséquent, le comprimé matriciel d'oxycodone HCl dosé à 40mg contenant la gomme xanthane comme seul agent gélifiant ne permet pas d'empêcher l'extraction d'actif dans l'éthanol à 96%.

### Exemple 2: Exemple comparatif dépourvu des éléments essentiels de l'invention de préparation d'un comprimé matriciel d'oxycodone HCl dosé à 40mg contenant un carbomère comme seul agent gélifiant mais pas de gomme xanthane

| composants | mg/ comprimé | pourcentage |
|---|---|---|
| 1.Oxycodone granulé | 43.55 | 28.46 |
| 2. Kollidon SR® (PVA/PVP 80:20) | 81.19 | 53.07 |
| 3.Carbomère (Carbopol®) | 27.00 | 17.65 |
| 4.Syloïd | 0.36 | 0.24 |
| 5.Stéarate de magnésium | 0.90 | 0.59 |
| total | 153.00 | 100.00 |
| Dureté | 417 N | |
| Résistance à la rupture | 9.7 MPa | |

Dans l'exemple 2, le mélange est préparé de manière identique à l'exemple 1. Les comprimés obtenus à une force de 14kN ont une dureté supérieure à 400N correspondant à une résistance à la rupture supérieure à 9 MPa.

Pourcentages extraits dans différents milieux à partir des comprimés obtenus selon l'exemple 2

| Eau purifiée | HCl 0.1N | Ethanol 96% |
|---|---|---|
| 53% | 62% | 21% |

Lorsque le comprimé préparé selon l'exemple 2 est broyé puis repris avec un volume de 10mL d'Ethanol 96%, la quantité d'oxycodone HCl recueillie après filtration est 21% de la dose initiale. Par contre le pourcentage dans les milieux aqueux est supérieur à 50% de la dose.

Par conséquent, le comprimé matriciel d'oxycodone HCl dosé à 40mg contenant un carbomère comme seul agent gélifiant ne permet pas d'empêcher l'extraction d'actif dans l'HCl 0.1N%.

### Exemple 3 : Exemple comparatif dépourvu des éléments essentiels de l'invention, basé sur l'Oxycontin®, comprimé dosé à 40mg d'Oxycodone HCl

Pourcentages extraits dans différents milieux à partir des comprimés d'Oxycontin®

| Eau purifiée | Ethanol 96% |
|---|---|
| 25% | 39% |

Lorsque le comprimé est coupé puis broyé et repris avec un volume de 10mL d'Ethanol 96%, la quantité d'oxycodone HCl recueillie après filtration est 39% de la dose initiale. La quantité recueillie après filtration dans l'eau purifiée est 25% de la dose initiale.

Par conséquent, ces résultats montrent que l'extraction d'actif est possible. Ces résultats sont améliorés par l'invention.

### Exemple 4 : comprimé matriciel d'oxycodone HCl dosé à 40mg selon l'invention contenant de la gomme xanthane et un carbomère en tant qu'agents gélifiants.

| composants | mg/ comprimé | pourcentage |
|---|---|---|
| 1.Oxycodone granulé | 43.68 | 25.69 |
| 2. Kollidon SR® (PVA/PVP 80:20) | 74.13 | 43.61 |
| 3.Gomme Xanthane | 25.50 | 15.00 |
| 4.Carbomère (Carbopol®) | 25.50 | 15.00 |
| 5.Syloïd | 0.34 | 0.20 |
| 6.Stéarate de magnésium | 0.85 | 0.50 |
| total | 170.00 | 100.00 |
| Dureté | 483 N | |
| Résistance à la rupture | 10.5 N | |

Dans l'exemple 4, les composants 1 à 5 sont tamisés sur 500µm puis mélangés au Turbula 10 min. Le stéarate de magnésium est ensuite ajouté et le temps de lubrification de 1 min. Le mélange est ensuite comprimé directement sur presse rotative Sviac, équipée avec des poinçons ronds de diamètre 7mm, à une force de 16kN. La dureté qui en résulte est supérieure à 400N correspondant à une résistance à la rupture supérieure à 10MPa.

Pourcentages extraits dans différents milieux à partir des comprimés obtenus selon l'exemple 3

| Eau purifiée | HCl 0.1N | Ethanol 96% |
|---|---|---|
| 0% | 8% | 23% |

Pourcentages extraits dans différents milieux à partir des comprimés obtenus selon l'exemple 3

Lorsque le comprimé préparé selon l'exemple 4 est broyé puis repris avec un volume de 10mL d'Ethanol 96%, la quantité d'oxycodone HCl recueillie après filtration est 23% de la dose initiale. La quantité recueillie dans les milieux aqueux est inférieure à 10%.

### Exemple 5 : Comprimé matriciel d'oxycodone HCl dosé à 40mg selon l'invention contenant de la gomme Xanthane, un carbomère et du bicarbonate de sodium

| Composants | mg/ comprimé | pourcentage |
|---|---|---|
| 1.Oxycodone granulé | 43.55 | 22.92 |
| 2. Kollidon SR® (PVA/PVP 80:20) | 88.12 | 46.38 |
| 3.Gomme Xanthane | 30.00 | 15.79 |
| 4.Carbomère (Carbopol®) | 22.50 | 11.84 |
| 5.NaHCO3 | 4.50 | 2.37 |
| 6.Syloïd | 0.38 | 0.20 |
| 7.Stéarate de magnésium | 0.95 | 0.50 |
| total | 190.00 | 100.00 |
| Dureté | 474 N | |
| Résistance à la rupture | 9.3 MPa | |

Dans l'exemple 5, les composants 1 à 6 sont tamisés sur 500µm puis mélangés au Turbula 10 min. Le stéarate de magnésium est ensuite ajouté et mélangé 1min supplémentaire pour lubrification. Le mélange est ensuite comprimé directement sur presse rotative Sviac, équipée avec des poinçons ronds de diamètre 7mm, à une force de 13kN. La dureté qui en résulte est supérieure à 400N correspondant à une résistance à la rupture supérieure à 9MPa.

| Eau purifiée | HCl 0.1N | Ethanol 96% |
|---|---|---|
| 0% | 0% | 30% |

Pourcentages extraits dans différents milieux à partir des comprimés obtenus selon l'exemple 4

Lorsque le comprimé est broyé puis repris avec un volume de 10mL d'Ethanol 96%, la quantité d'oxycodone recueillie après filtration est 30% de la dose initiale. La quantité recueillie après filtration dans les milieux aqueux testés est de 0%.

Par ailleurs, quand la solution n'est pas filtrée et qu'elle est introduite directement dans une seringue contenant une aiguille de 20 gauge, cette solution possède une telle viscosité qu'elle ne passe pas à travers l'aiguille.

Par ailleurs, les profils de dissolution générés dans les milieux tampon pH 1.2 et pH 6.8 ne montrent pas de pH-dépendance de la composition.

### Exemple 6 : Comprimé matriciel d'oxycodone HCl dosé à 40mg selon l'invention contenant de la gomme Xanthane, un carbomère et de l'acide citrique

| composants | mg/comprimé | pourcentage |
|---|---|---|
| 1.Oxycodone granulé | 43.55 | 22.92 |
| 2. Kollidon SR® (PVA/PVP 80:20) | 88.12 | 46.38 |
| 3.Gomme Xanthane | 30.00 | 15.79 |
| 4.Carbomère (Carbopol®) | 22.50 | 11.84 |
| 5.Acide citrique | 4.50 | 2.37 |
| 6.Syloïd | 0.38 | 0.20 |
| 7.Stéarate de magnésium | 0.95 | 0.50 |
| total | 190.00 | 100.00 |
| Dureté | 440 N | |
| Résistance à la rupture | 8.7 MPa | |

L'exemple 6 est préparé de manière identique à l'exemple 5. La dureté qui en résulte est supérieure à 400N correspondant à une résistance à la rupture supérieure à 8MPa.

Pourcentages extraits dans différents milieux à partir des comprimés obtenus selon l'exemple 5

| Eau purifiée | HCl 0.1N | Ethanol 96% |
|---|---|---|
| 0% | 0% | 20% |

Lorsque le comprimé est broyé puis repris avec un volume de 10mL d'Ethanol 96%, la quantité d'oxycodone recueillie après filtration est 20% de la dose initiale. La quantité recueillie après filtration dans les milieux aqueux testés est de 0%.

Par ailleurs, quand la solution n'est pas filtrée et qu'elle est introduite directement dans une seringue contenant une aiguille de 20 gauge, cette solution possède une telle viscosité qu'elle ne passe pas à travers l'aiguille.

Par ailleurs, les profils de dissolution générés dans les milieux tampon pH 1.2 et pH 6.8 ne montrent pas de pH-dépendance de la composition.

### Exemple 7 : Comprimé matriciel d'hydromorphone dosé à 32mg selon l'invention contenant de la gomme Xanthane, un carbomère et de l'acide citrique

| composants | mg/ comprimé | pourcentage |
|---|---|---|
| 1.Hydromorphone | 32.00 | 25.60 |
| 2. Kollidon SR® (PVA/PVP 80:20) | 35.12 | 28.10 |
| 3.Gomme Xanthane | 30.00 | 24.00 |
| 4. Carbomère (Carbopol®) | 22.50 | 18.00 |
| 5.Acide citrique | 4.50 | 3.60 |
| 6.Syloïd | 0.25 | 0.20 |
| 7.Stéarate de magnésium | 0.63 | 0.50 |
| total | 125.00 | 100.00 |
| Dureté | 268 N | |
| Résistance à la rupture | 7.7 MPa | |

L'exemple 7 est préparé de manière identique à l'exemple 6. La dureté qui en résulte correspond à une résistance à la rupture supérieure à 7MPa.

Pourcentages extraits dans différents milieux à partir des comprimés obtenus selon l'exemple 6

| Eau purifiée | HCl 0.1N | Ethanol 96% |
|---|---|---|
| 0% | 6% | 20% |

Lorsque le comprimé est broyé puis repris avec un volume de 10mL d'Ethanol 96%, la quantité d'hydromorphone recueillie après filtration est 20% de la dose initiale. La quantité recueillie après filtration dans les milieux aqueux testés inférieure à 10%.

### Exemple 8 : Comprimé multicouche d'oxycodone HCl dosé à 40mg selon l'invention contenant de la gomme Xanthane, un carbomère et de l'acide citrique

| **composants** | **mg / comprimé** | **%** |
|---|---|---|
| Oxycodone HCl granulé | 43,55 | 16,40 |
| Kollidon SR® (PVA/PVP 80:20) | 88,12 | 33,19 |
| Gomme Xanthane | 30,00 | 11,30 |
| Syloïd | 0,32 | 0,12 |
| Stéarate de Magnésium | 0,80 | 0,30 |
| **TOTAL couche 1** | **162,79** | **61,32** |
| Kollidon SR® (PVA/PVP 80:20) | 30,00 | 11,30 |
| Gomme Xanthane | 15,00 | 5,65 |
| Syloïd | 0,10 | 0,04 |
| Stéarate de Magnésium | 0,22 | 0,08 |
| **TOTAL couche 2** | **45,32** | **17,07** |
| Carbomère (Carbopol®) | 22,50 | 8,47 |
| Acide citrique | 4,50 | 1,69 |
| Kollidon SR® (PVA/PVP 80:20) | 30,00 | 11,30 |
| Syloïd | 0,11 | 0,04 |
| Stéarate de Magnésium | 0,28 | 0,11 |
| **TOTAL couche 3** | **57,39** | **21,62** |
| **TOTAL** | **265,50** | **100.00** |
| **Dureté** | **430 N** | |
| **Résistance à la rupture** | **6.7MPa** | |

Dans l'exemple 8, le mélange de chaque couche est préparé séparément de la même manière que les mélanges dans les exemples précédents.

Les comprimés obtenus sur une presse rotative équipée avec des poinçons de diamètre 8.5mm ont une dureté finale supérieure à 400N correspondant à une résistance à la rupture supérieure à 6MPa.

Pourcentages extraits dans différents milieux à partir des comprimés obtenus selon l'exemple 7

| Eau purifiée | HCl 0.1N | Ethanol 96% |
|---|---|---|
| 0% | 0% | 26% |

Lorsque le comprimé multicouche est broyé puis repris avec un volume de 10ml d'Ethanol 96%, la quantité d'oxycodone HCl recueillie après filtration est de 26% de la dose initiale. La quantité recueillie après filtration dans les milieux aqueux testés est de 0%.

En conclusion, la présence de gomme xanthane et de Carbopol est essentielle pour limiter la décharge de principe actif en milieu aqueux ou hydroalcoolique.

### Exemple 9 : micro-comprimés de sulfate de morphine : association de 2 populations différentes de micro-comprimés dosés unitairement à 10 mg de sulfate de morphine contenant de la gomme Xanthane, un carbomère et un stabilisateur de pH.

**Population 1**

| **Composition** | mg/cp | % |
|---|---|---|
| 1. Sulfate de Morphine | 10,00 | 40,00 |
| 2. Kollidon SR® (PVA/PVP 80:20) | 10,50 | 42,00 |
| 3. Gomme Xanthane | 4,00 | 16,00 |
| 4. Cellulose microcristalline (Avicel PH102) | 0,13 | 0,52 |
| 5. Syloïd 244 FP | 0,12 | 0,48 |
| 6. Stéarate de Mg | 0,25 | 1,00 |
| Total | 25,00 | 100,00 |
| Dureté | 111N | |
| Résistance à la rupture | 7.3MPa | |

**Population 2**

| **Composition** | **mg/cp** | **%** |
|---|---|---|
| 1. Carbopol® 71G | 18,500 | 74,0 |
| 2. Ac citrique ou Bicarbonate de Na | 3,700 | 14,8 |
| 3. Cellulose microcristalline | 2,550 | 10,2 |
| 4. Syloïd 244 FP | 0,125 | 0,5 |
| 5. Stéarate de Mg | 0,125 | 0,5 |
| **Total** | 25,000 | 100,0 |

Dans l'exemple 9, la population 1 de micro-comprimés est préparée de la façon suivante : les composants 1 à 4 sont tamisés sur 500µm puis mélangés au Turbula 10 min. Le stéarate de magnésium est ensuite ajouté et mélangé 1min supplémentaire pour lubrification. Le mélange est ensuite comprimé directement sur presse rotative Sviac, équipée avec des poinçons ronds de diamètre 3mm, à une force de 2kN. La dureté qui en résulte est supérieure à 100N correspondant à une résistance à la rupture supérieure à 7MPa.

La population 2 est préparée de manière similaire : les composants 1 à 4 sont tamisés avant d'être mélangés au Turbula 5 min. Le stéarate de magnésium est ensuite ajouté et mélangé 1 min supplémentaire pour lubrification.

Les deux populations sont associées de la manière suivante : 6 micro-comprimés de la population 1 et 2 micro-comprimés de la population 2 ce qui conduit à la composition unitaire (contenu d'une gélule) suivante :

| *Composition* | 6 microcomprimés de la polupation 1 + 2 micro-comprimés de la population 2 | |
|---|---|---|
| | mg | % |
| Sulfate de Morphine | 60,0 | 30,0 |
| Kollidon SR® (PVA/PVP 80:20) | 63,0 | 31,5 |
| Gomme Xanthane | 24,0 | 12,0 |
| Carbopol 71G | 37,0 | 18,5 |
| Stabilisateur de pH | 7,4 | 3,7 |
| Avicel PH102 | 5,8 | 2,9 |
| Syloïd 244 FP | 1,0 | 0,5 |
| Stéarate de magnésium | 1,8 | 0,9 |
| Total | 200,0 | 100,0 |

Pourcentages extraits dans différents milieux à partir des micro-comprimés obtenus selon l'exemple 9 lorsque le stabilisateur de pH est l'acide citrique :

| Eau purifiée | HCl 0.1N | Ethanol 96% |
|---|---|---|
| 2% | 11% | 6% |

Pourcentages extraits dans différents milieux à partir des micro-comprimés obtenus selon l'exemple 9 lorsque le stabilisateur de pH est le bicarbonate de sodium :

| Eau purifiée | HCl 0.1N | Ethanol 96% |
|---|---|---|
| 0% | 10% | 6% |

Lorsque les micro-comprimés de l'exemple 9 sont broyés puis repris avec un volume de 10ml d'Ethanol 96%, la quantité de sulfate de morphine recueillie après filtration est inférieure à 10% de la dose initiale, que ce soit dans l'eau ou dans l'Ethanol 96%, et ce pour les deux stabilisateurs de pH. La quantité recueillie après filtration dans l'HCl 0.1N est de l'ordre de 10% pour les deux stabilisateurs de pH.

### Exemple 10 : micro-comprimés de sulfate de morphine : association de 2 populations différentes par le ratio des composants.

Dans l'exemple 10, deux populations de composition identique aux populations 1 et 2 de l'exemple 9, sont associées de la manière suivante : 10 micro-comprimés de la population et 1 micro-comprimé de la population 2 ce qui conduit à la composition unitaire (contenu d'une gélule) suivante :

| Composition | | | 10 microcomprimés de la polupation 1 + 1 micro comprimé de la population 2 | |
|---|---|---|---|---|
| | | | mg | % |
| Sulfate de Morphine | | | 100,0 | 36,4 |
| Kollidon SR® (PVA/PVP 80:20) | | | 105,0 | 38,2 |
| Gomme Xanthane | | | 40,0 | 14,5 |
| Carbopol 71G | | | 18,5 | 6,7 |
| Stabilisateur de pH | | | 3,7 | 1,3 |
| Avicel PH102 | | | 3,9 | 1,4 |
| Syloïd 244 FP | | | 1,3 | 0,5 |
| Stéarate de magnésium | | | 2,6 | 1,0 |
| Total | | | 275,0 | 100,0 |

Pourcentages extraits dans différents milieux à partir de l'association des micro-comprimés obtenus selon l'exemple 10 lorsque le stabilisateur de pH est l'acide citrique :

| Eau purifiée | HCl 0.1N | Ethanol 96% |
|---|---|---|
| 0% | 3% | 7% |

Pourcentages extraits dans différents milieux à partir de l'association des micro-comprimés obtenus selon l'exemple 10 lorsque le stabilisateur de pH est le bicarbonate de sodium :

| Eau purifiée | HCl 0.1N | Ethanol 96% |
|---|---|---|
| 0% | 2% | 6% |

Lorsque les micro-comprimés de l'exemple 9 sont broyés puis repris avec un volume de 10ml d'Ethanol 96%, la quantité de morphine sulfate recueillie après filtration est inférieure ou égale à 10% de la dose initiale dans les trois milieux.

## Revendications

1. Composition pharmaceutique orale sous forme de comprimé à libération prolongée comprenant :
- au moins un principe actif susceptible d'être détourné à des fins de toxicomanie,
- un mélange d'acétate de polyvinyle et de polyvinylpyrrolidone,
- de la gomme xanthane,
- un carbomère.

2. Composition pharmaceutique selon la revendication 1, **caractérisée en ce que** le ou lesdits principes actifs sont choisis dans le groupe comprenant les psychotropes, les antipsychotiques, les tranquillisants, les hypnotiques, les analgésiques et les anxiolytiques.

3. Composition pharmaceutique selon les revendications 1 et 2, **caractérisée en ce que** le ou lesdits principes actifs appartiennent au groupe comprenant la morphine, l'oxycodone, l'hydrocodone, l'hydromorphone, l'oxymorphone, le tramadol, la méthadone, la codéine, le fentanyl et la buprénorphine, les cannabinoïdes, la cocaïne, les amphétamines, leurs sels et leurs dérivés pharmaceutiquement acceptables.

4. Composition pharmaceutique selon l'une quelconque des revendications 1 à 3, **caractérisée en ce que** le mélange d'acétate de polyvinyle et de polyvinylpyrrolidone représente de 10 à 80 % en poids du poids total de la composition, de préférence de 20 à 60 % en poids du poids total de la composition.

5. Composition pharmaceutique selon l'une quelconque des revendications 1 à 4, **caractérisée en ce que** le ratio d'acétate de polyvinyle et de polyvinylpyrrolidone est compris entre (95:5) et (70:30), de préférence est de 80:20.

6. Composition pharmaceutique selon l'une quelconque des revendications 1 à 5, **caractérisée en ce que** la gomme xanthane représente de 1 à 88% en poids du poids total de la composition, de préférence de 5 à 30 % en poids du poids total de la composition.

7. Composition pharmaceutique selon l'une quelconque des revendications 1 à 6, **caractérisée en ce que** le carbomère représente de 1 à 88 % en poids du poids total de la composition, de préférence de 5 à 40 % en poids du poids total de la composition.

8. Composition pharmaceutique selon les revendications 1 à 7, **caractérisée en ce qu'**elle comprend un agent stabilisateur de pH, de préférence en une quantité de 0,1 à 30 % en poids du poids total de la composition.

9. Composition pharmaceutique selon l'une quelconque des revendications 1 à 8, **caractérisée en ce qu'**elle comprend en outre au moins une ou plusieurs des substances (a) à (e) qui suivent ou leur mélange :
a) une substance qui irrite les voies nasale et/ou pharyngée,
b) une substance émétique,
c) un colorant aversif,
d) une substance au goût amer,
e) un antagoniste du ou des principe(s) actif(s) susceptible(s) d'être détourné(s) à des fins de toxicomanie, de préférence la naloxone ou la naltrexone ou un de leurs sels pharmaceutiquement acceptables.

10. Composition pharmaceutique selon l'une quelconque des revendications 1 à 9, **caractérisée en ce qu'**elle comporte un enrobage externe, de préférence constitué d'au moins un polymère retard choisi dans le groupe des dérivés d'éthylcellulose et des polymères méthacryliques.

11. Composition pharmaceutique selon l'une quelconque des revendications 1 à 10, **caractérisée en ce qu'**elle se présente sous la forme de comprimé matriciel, de comprimé multicouche ou de micro-comprimés.

12. Composition pharmaceutique selon l'une quelconque des revendications 1 à 11 pour son utilisation comme médicament pour lutter contre le détournement à des fins de toxicomanie par voie injectable, nasale ou orale.

13. Composition pharmaceutique selon l'une quelconque des revendications 1 à 11 pour son utilisation comme médicament pour lutter contre le mésusage involontaire.

14. Composition pharmaceutique selon l'une quelconque des revendications 1 à 11 pour son utilisation comme médicament tel que décrit dans les revendications 12 ou 13, ledit médicament est susceptible d'être administré une fois par jour.

15. Composition pharmaceutique selon l'une quelconque des revendications 1 à 11 pour son utilisation comme médicament tel que décrit dans les revendications 12 ou 13, ledit médicament est susceptible d'être administré deux fois par jour.

16. Procédé de fabrication des comprimés matriciels selon la revendication 11, **caractérisé en ce qu'**il comporte les étapes suivantes :
- mélange du ou des principes actifs avec le ou les excipients de la matrice,
- éventuellement granulation et
- compression dudit mélange dans des conditions choisies de manière à ce que ledit comprimé présente une résistance à l'écrasement d'au moins 4 MPa, avantageusement d'au moins 6 MPa.

## Patentansprüche

1. Orale pharmazeutische Zusammensetzung in Form einer Tablette mit verzögerter Freisetzung, umfassend:
- zumindest einen Wirkstoff, der zum Drogenmissbrauch zweckentfremdet werden kann,
- eine Mischung aus Polyvinylacetat und Polyvinylpyrrolidon,
- Xanthangummi,
- ein Carbomer.

2. Pharmazeutische Zusammensetzung nach Anspruch 1, **dadurch gekennzeichnet, dass** der oder die Wirkstoff (e) aus der Gruppe umfassend Psychopharmaka, Antipsychotika, Beruhigungsmittel, Hypnotika, Schmerzmittel und Anxiolytika ausgewählt sind.

3. Pharmazeutische Zusammensetzung nach den Ansprüchen 1 und 2, **dadurch gekennzeichnet, dass** der oder die Wirkstoff(e) zu der Gruppe gehören, die Morphium, Oxycodon, Hydrocodon, Hydromorphon, Oxymorphon, Tramadol, Methadon, Codein, Fentanyl und Buprenorphin, Cannabinoide, Kokain, Amphetamine, deren pharmazeutisch annehmbare salze und Derivate, umfasst.

4. Pharmazeutische Zusammensetzung nach einem beliebigen der vorstehenden Ansprüche 1 bis 3, **dadurch gekennzeichnet, dass** die Mischung aus Polyvinylacetat und Polyvinylpyrrolidon 10 bis 80 Gew.-% des Gesamtgewichts der Zusammensetzung, vorzugsweise 20 bis 60 Gew.-% des Gesamtgewichts der Zusammensetzung, ausmacht.

5. Pharmazeutische Zusammensetzung nach einem beliebigen der vorstehenden Ansprüche 1 bis 4, **dadurch gekennzeichnet, dass** das Verhältnis von Polyvinylacetat und Polyvinylpyrrolidon zwischen (95 : 5) und (70 : 30), vorzugsweise bei 80 : 20, liegt.

6. Pharmazeutische Zusammensetzung nach einem beliebigen der vorstehenden Ansprüche 1 bis 5, **dadurch gekennzeichnet, dass** Xanthangummi 1 bis 88 Gew.-% des Gesamtgewichts der Zusammensetzung, vorzugsweise 5 bis 30 Gew.-% des Gesamtgewichts der Zusammensetzung ausmacht.

7. Pharmazeutische Zusammensetzung nach einem beliebigen der vorstehenden Ansprüche 1 bis 6, **dadurch gekennzeichnet, dass** das Carbomer 1 bis 88 Gew.-% des Gesamtgewichts der Zusammensetzung, vorzugsweise 5 bis 40 Gew.-% des Gesamtgewichts der Zusammensetzung ausmacht.

8. Pharmazeutische Zusammensetzung nach einem beliebigen der vorstehenden Ansprüche 1 bis 7, **dadurch gekennzeichnet, dass** sie einen pH-Stabilisator, vorzugsweise in einer Menge von 0,1 bis 30 Gew.-% des Gesamtgewichts der Zusammensetzung, enthält.

9. Pharmazeutische Zusammensetzung nach einem beliebigen der vorstehenden Ansprüche 1 bis 8, **dadurch gekennzeichnet, dass** sie ferner zumindest eine oder mehrere der folgenden Substanzen (a) bis (e) oder deren Mischung enthält:
a) eine Substanz, die die Nasen- und/oder Rachenwege reizt,
b) ein Brechmittel,
c) einen aversiven Farbstoff,
d) eine bitter schmeckende Substanz,
e) einen Antagonisten des oder der Wirkstoff(e), der oder die zum Zwecke des Drogenmissbrauchs zweckentfremdet werden kann/können, vorzugsweise Naloxon oder Naltrexon oder ein pharmazeutisch annehmbares Salz davon.

10. Pharmazeutische Zusammensetzung nach einem beliebigen der vorstehenden Ansprüche 1 bis 9, **dadurch gekennzeichnet, dass** sie eine äußere Beschichtung aufweist, die vorzugsweise aus zumindest einem verzögerten Polymer gebildet ist, das aus der Gruppe der Ethylcellulosederivate und Methacrylpolymere gewählt wird.

11. Pharmazeutische Zusammensetzung nach einem beliebigen der vorstehenden Ansprüche 1 bis 10, **dadurch gekennzeichnet, dass** sie in Form einer Matrixtablette, einer Mehrschichttablette oder Mikrotabletten vorliegt.

12. Pharmazeutische Zusammensetzung nach einem beliebigen der vorstehenden Ansprüche 1 bis 11 zur Verwendung als Medikament zur Bekämpfung der Zweckentfremdung bei injizierbarem, nasalem oder oralem Drogenmissbrauch.

13. Pharmazeutische Zusammensetzung nach einem beliebigen der vorstehenden Ansprüche 1 bis 11 zur Verwendung als Medikament zur Bekämpfung von unbeabsichtigtem Missbrauch.

14. Pharmazeutische Zusammensetzung nach einem beliebigen der vorstehenden Ansprüche 1 bis 11 zur Verwendung als Medikament wie in den Ansprüchen 12 oder 13 beschrieben, wobei das Medikament einmal täglich verabreicht werden kann.

15. Pharmazeutische Zusammensetzung nach einem beliebigen der vorstehenden Ansprüche 1 bis 11 zur Verwendung als Medikament wie in den Ansprüchen 12 oder 13 beschrieben, wobei das Medikament zweimal täglich verabreicht werden kann.

16. Verfahren zur Herstellung der Matrixtabletten nach Anspruch 11, **dadurch gekennzeichnet, dass** es die folgenden Schritte umfasst:
- Mischen des/der Wirkstoffe(s) mit dem/den Hilfsstoff(en) der Matrix,
- eventuell Granulierung und
- Verdichtung der Mischung unter ausgewählten Bedingungen, so dass die Tablette einen Druckverformungswiderstand von zumindest 4 MPa, vorteilhafterweise von zumindest 6 MPa aufweist.

## Claims

1. An oral pharmaceutical composition in the form of a sustained-release tablet comprising:
- at least one active ingredient likely to be misused for drug addiction purposes;
- a mixture of polyvinyl acetate and polyvinylpyrrolidone,
- xanthan gum,
- a carbomer.

2. The pharmaceutical composition according to claim 1, **characterized in that** the said active ingredient(s) are selected from the group comprising psychotropics, antipsychotics, tranquillizers, hypnotics, analgesics and anxiolytics.

3. The pharmaceutical composition according to claims 1 and 2, **characterized in that** the said active ingredient(s) belong to the group comprising morphine, oxycodone, hydrocodone, hydromorphone, oxymorphone, tramadol, methadone, codeine, fentanyl and buprenorphine, cannabinoids, cocaine, amphetamines, their pharmaceutically acceptable salts and derivatives.

4. The pharmaceutical composition according to any of claims 1 to 3, **characterized in that** the mixture of polyvinyl acetate and polyvinylpyrrolidone represents 10 to 80 % by weight of the total weight of the composition, preferably 20 to 60 % by weight of the total weight of the composition.

5. The pharmaceutical composition according to any of claims 1 to 4, **characterized in that** the ratio of polyvinyl acetate and polyvinylpyrrolidone is between (95:5) and (70:30), and is preferably 80:20.

6. The pharmaceutical composition according to any of claims 1 to 5, **characterized in that** the xanthan gum represents 1 to 88% by weight of the total weight of the composition, preferably 5 to 30 % by weight of the total weight of the composition.

7. The pharmaceutical composition according to any of claims 1 to 6, **characterized in that** the carbomer represents 1 to 88 % by weight of the total weight of the composition, preferably 5 to 40 % by weight of the total weight of the composition.

8. The pharmaceutical composition according to claims 1 to 7, **characterized in that** it comprises a pH stabilizing agent, preferably in an amount of 0.1 to 30 % by weight of the total weight of the composition.

9. The pharmaceutical composition according to any of claims 1 to 8, **characterized in that** it further comprises at least one or more of the following substances (a) to (e) or a mixture thereof:
a) a substance which irritates the nasal and/or pharyngeal pathways;
b) an emetic substance;
c) an aversive colouring;
d) a substance of bitter taste;
e) an antagonist of the active ingredient(s) likely to be misused for drug addiction purposes, preferably naloxone or naltrexone or one of the pharmaceutically acceptable salts thereof.

10. The pharmaceutical composition according to any of claims 1 to 9, **characterized in that** it has an outer coating, preferably formed of at least one sustained-release polymer selected from the group of ethylcellulose derivatives and methacrylic polymers.

11. The pharmaceutical composition according to any of claims 1 to 10, **characterized in that** it is in the form of a matrix tablet, multilayer tablet or micro-tablets.

12. The pharmaceutical composition according to any of claims 1 to 11 for use thereof as medication to combat misuse via injectable, nasal or oral route for drug addiction purposes.

13. The pharmaceutical composition according to any of claims 1 to 11 for use thereof as medication to combat accidental misuse.

14. The pharmaceutical composition according to any of claims 1 to 11 for use thereof as medication as described in claim 12 or 13, said medication is able to be administered once daily.

15. The pharmaceutical composition according to any of claims 1 to 11 for use thereof as medication as described in claim 12 or 13, said medication is able to be administered twice daily.

16. A method for manufacturing matrix tablets according to claim 11, **characterized in that** it comprises the following steps:
- mixing the active ingredient(s) with the excipient(s) of the matrix;
- optional granulation; and
- compressing the said mixture under conditions chosen so that the said tablet has a crush resistance of at least 4 MPa, advantageously at least 6 MPa.
